# EUROPEAN PATENT APPLICATION

(11) **EP 4 796 168 A1**
(43) Date of publication of application: **26.08.2026**
(21) Application number: 25159060.0
(22) Date of filing: 20.02.2025
(51) Int. Cl.: A61N 5/10

(54) **FIELD-TO-TARGET ASSIGNMENT IN RADIOTHERAPY TREATMENT PLANNING**

(71) Applicant: Siemens Healthineers International AG, 6312 Steinhausen (CH)
(72) Inventor: VALENZUELA, Daniel, 0440 Helsinki (FI); TALLINEN, Tuomas, 02600 Espoo (FI)
(74) Representative: Mathisen & Macara LLP

(57) **Abstract**

A method (900) for generating and optimising a radiotherapy treatment plan for treating a plurality of targets (822-826) using a plurality of fields (802-806), each field (802-806) comprising an optimisable set of control points and each control point comprising a set of beam geometry parameters; wherein: the method (900) comprises determining (908) a plurality of field-target assignments (812-816) for the plan by assigning each of the plurality of fields (802-806) to a respective non-empty subset of the plurality of targets (822-826); at least one field (802-806) is assigned to each target; and at least one field (802-806) is assigned to a non-empty proper subset of the plurality of targets.

## Description

### TECHNICAL FIELD

This invention relates to radiotherapy treatment planning, and in particular, to methods for generating or optimising radiotherapy treatment plans that reduce or eliminate dose bridging or the irradiation of healthy tissue located between multiple tumours.

### BACKGROUND

The use of energy to treat medical conditions comprises a known area of prior art endeavour. For example, radiation therapy comprises an important component of many treatment plans for reducing or eliminating unwanted tumours. Unfortunately, applied energy does not inherently discriminate between unwanted material and adjacent tissues, organs, or the like that are desired or even critical to continued survival of the patient (such adjacent tissues may be referred to as "organs at risk", OARs). As a result, energy such as radiation is ordinarily applied in a carefully administered manner to at least attempt to restrict the energy to a given target volume. A so-called radiation treatment plan often serves in the foregoing regards.

A radiation treatment plan typically comprises specified values for each of a variety of treatment-platform "machine" parameters during each of a plurality of sequential fields. For example, machine parameters of a radiotherapy system may include the angle of irradiation of the treatment beam, or the configuration of the leaves of a multileaf collimator.

Treatment plans for radiation treatment sessions are often automatically generated through a so-called optimisation process. As used herein, "optimisation" will be understood to refer to improving a candidate treatment plan without necessarily ensuring that the optimised result is, in fact, the singular best solution. Such optimisation often includes automatically adjusting one or more physical treatment machine parameters (often while observing one or more corresponding limits in these regards) and mathematically calculating a likely corresponding treatment result (such as a level of dosing) to identify a given set of treatment parameters that represent a good compromise between the desired therapeutic result and avoidance of undesired collateral effects.

In some cases, optimisation proceeds as a function of multiple different criteria or clinical objectives. This may involve the use of a cost function comprising a mathematical model that attempts to balance conflicting clinical goals or objectives to produce a treatment plan with the least "cost". Clinical goals generally take the form of a particular metric being required to be greater or smaller than a threshold value. For example, a clinical goal or objective may require that a certain percentage of the volume of an OAR receives less than a certain amount of dose. As a further example, a clinical goal or objective may require that a certain percentage of the planning target volume (PTV) receives at least a certain amount of dose. A cost function may be a sum of terms relating to deviations from the clinical goals.

A challenge in plan optimisation for cancer patients with multiple tumours is the occurrence of "dose bridging". "Dose bridging" refers to radiation treatment that results in healthy tissue located between multiple targets receiving a higher dose than what is desirable.

For example, dose bridging may occur in Volumetric Modulated Arc Therapy (VMAT) or in Intensity Modulated Radiation Therapy (IMRT).

In VMAT, radiation is delivered to a patient while the radiation source rotates around the patient in one or more arcs. An "arc" may either be a "full" arc which rotates 360° or a "partial" arc that rotates less than 360°. During continuous rotation, the dose rate, the shape of the beam and the speed of the gantry are dynamically modulated so that the radiation optimally conforms to one or more targets while avoiding exposure to surrounding healthy tissue ("organs at risk", OARs).

In IMRT, radiation is delivered from multiple static angles. Radiation is delivered in discrete steps, where the radiotherapy machine rotates to different fixed angles and delivers radiation at each angle.

In VMAT and IMRT, the notion of a "field" refers to a set of optimisable control points, each control point relating to a set of beam geometry parameters. Beam geometry parameters may include treatment isocenter, couch position or collimator angle. A further beam geometry may be gantry angle (in IMRT) or start-stop gantry angle range (in VMAT). In VMAT, a field comprises a single arc having a set of optimisable control points. In IMRT, a field refers to a single radiation beam directed at a patient from a particular angle or control point. Dose bridging may occur in either VMAT or IMRT plans where a field irradiates multiple physically separate targets resulting in radiation being delivered to healthy tissue in between the physically separate targets.

There are existing approaches that aim to reduce the dose bridging. Some known solutions in VMAT therapy include the use of five partial VMAT arcs at predetermined positions, and a cost function that includes a dedicated normal tissue objective that is specially tailored to reduce dose bridging. The rationale of existing solutions is that by providing more directions from which therapeutic dose can enter, the plan optimiser can meet the objectives of both the targets and the normal tissue, effectively reducing dose bridging.

However, in prior art VMAT and IMRT planning, the optimisation process takes an approach where all the fields aim to treat all the targets, equally. This results in a non-optimal reduction of dose-bridging.

### SUMMARY OF THE INVENTION

In accordance with a first aspect of the invention, there is provided a method for generating and optimising a radiotherapy treatment plan for treating a plurality of targets using a plurality of fields, as defined by claim 1.

In accordance with a second aspect of the invention, there is provided a radiation treatment planning computer system as defined by claim 14.

In accordance with a third aspect of the invention, there is provided a computer program product comprising a non-transitory, computer readable storage medium encoded with instructions operable for execution by a processor, as defined by claim 15.

Optional features are defined by the dependent claims.

The first aspect of the invention comprises a method for generating and optimising a radiotherapy treatment plan for treating a plurality of targets using a plurality of fields, each field comprising an optimisable set of control points and each control point comprising a set of beam geometry parameters; wherein: the method comprises determining a plurality of field-target assignments for the plan by assigning each of the plurality of fields to a respective non-empty subset of the plurality of targets; at least one field is assigned to each target; and at least one field is assigned to a non-empty proper subset of the plurality of targets.

In the present disclosure, the term "field-target" assignment refers to the assignment of a field to a non-empty subset of the multiple targets.

Here, it is noted that some of the fields may be assigned to a non-proper subset of the targets, that is, some fields may be assigned to all the targets. However, at least one of the fields is assigned to a proper subset of the targets.

In an example, a plurality of fields may each be assigned to a respective proper subset of targets. In another example, a majority of fields (e.g. >50%) of fields may each be assigned to a respective proper subset of targets. As a further example, most (e.g. >90%) of fields may each be assigned to a respective proper subset of targets.

A proper subset may comprise a subset with any number of fewer elements than the full set of targets. In an example, a proper subset may comprise one fewer element than the full set of targets. In another example, a proper subset as used by the present method may comprise many (e.g. >30%) fewer elements than the full set of targets. In a further example, a proper subset as used by the present method may comprise more than 50% fewer elements than the full set of targets. In a still further example, a proper subset as used by the present method may comprise more than 90% fewer elements than the full set of targets.

Advantageously, by assigning a plurality of fields to a proper subset of the targets (rather than all fields treating all targets as in the prior art), there may be greater flexibility with regards to the portion of tissue that each field irradiates, so that irradiation that is incident upon healthy tissue may be reduced, resulting in an overall dose distribution that has reduced dose bridging.

The field-target assignment may be followed by further (conventional) dose-based optimisation.

In some cases, the number of fields (e.g. VMAT arcs) may be predetermined, prior to the treatment planning process. The number of fields may be optimised in a separate process prior to the present planning process.

Optionally, each of said plurality of field-target assignments for the plan is non-exclusive so that during optimisation of the plan, targets to which a field was not assigned may be planned to be treated by the field.

That is, during optimisation, if it proves advantageous or more efficient to irradiate a target from a field that was not assigned to the target, the optimiser will allow this. This may happen, for example, when treating a target to which a field is not assigned results in reduced dose bridging or irradiation of healthy tissue for the overall plan considering the dose delivered by all the fields.

### Determining cost of field-target assignments

Optionally, the method may further comprise calculating a cost associated with each possible assignment of each of the plurality of fields to a plurality of non-empty subsets of the plurality of targets, and wherein the plurality of field-target assignments for the plan comprises a set of assignments having a minimum sum of associated costs.

A cost associated with assigning a field to a subset of targets may be seen as a measure of the suitability of the field in treating the subset of targets.

Optionally, the cost associated with an assignment of a field to a non-empty subset of targets can be computed by one or more of:
(i) a cost of a plan generated by executing a plan optimiser using plan objectives while restricting the plan to said field and said non-empty subset of targets;
(ii) a quantification of an overlap between targets and organs at risk under the assignment;
(iii) a number of collimator leaves involved in the assignment;
(iv) an amount of healthy tissue exposed in a collimator aperture covering all targets in the non-empty subset of targets;
(v) distance from a source of radiation to the non-empty subset of targets; and
(vi) sum of leaves openings distances in a collimator aperture that conforms to the non-empty subset of targets.

The above list of ways by which to compute the cost associated with an assignment of a field to a non-empty subset of targets is exemplary and not exhaustive and variations and combinations of the above listed methods (i)-(vi) come within the scope of the present disclosure.

Method (i) above may relate to traditional objective function-based optimisation where machine parameters are iteratively changed, a resulting dose distribution is calculated, and a dose-based cost function then computes the cost of the treatment plan as defined by the machine parameters. The iterative modification of the machine parameters continues until the dose-based cost function is minimised. For example, the cost function could penalise deviations from dose-based goals such as minimum dose to a target, or maximum dose to an organ at risk. The present method (i) above for determining a cost associated with a field-target assignment may comprise using a cost function to determine the cost of a treatment plan which consists only of the field and targets of the assignment.

Method (ii) above considers the overlap between healthy tissue (OARs) and targets in the beam's eye view, for example as seen through a multileaf collimator aperture. The greater the extent to which healthy tissue overlaps the targets, the higher the cost associated with the corresponding field-target assignment.

Method (iii) above considers the extent to which the collimator leaves are required to move in order to realise a particular field-target assignment. Here, the greater the extent of collimator leaf movement, the greater the use of time resources and machine resources, and the greater the cost of the field-target assignment.

Method (iv) above considers the amount of healthy tissue exposed as a result of a collimator aperture of a field attempting to irradiate the targets to which the field has been assigned. The greater the amount of healthy tissue irradiated in this way, the greater the cost of the particular field-target assignment.

Method (v) above considers that the greater the distance of the subset of targets from the radiation source under a particular field-target assignment, the greater the cost of the field-target assignment.

Method (vi) considers that the greater the distance that the leaves need to travel to cover the non-empty subset of targets, the greater the cost of the corresponding field assignment.

### Beam geometry parameters

As noted above, a field comprises a set of control points each characterised by beam geometry parameters.

Optionally, the beam geometry parameters comprise one or more of:
multileaf collimator leaf positions;
collimator angle;
treatment isocenter;
couch position; and
start-stop gantry angle range.

Optionally, the beam geometry parameters are optimised to be static during a gantry rotation; or: the beam geometry parameters are optimised to be dynamic during a gantry rotation.

When the cost of a particular field-target assignment is being determined, the optimal set of beam geometry parameters for the field to irradiate the subset of targets of the assignment is determined. So, the cost is not determined with respect to an arbitrary field, but by the cost with respect to a field of optimal geometry to treat the subset of targets.

In VMAT, start-stop gantry angle range is one of the beam geometry parameters. When IMRT is used, rather than the start-stop gantry angle, the static gantry angle would be used as a beam geometry parameter instead.

In some implementations, one or more of the beam geometry parameters may be fixed, while the other parameters may be optimised or varied. For example, all the beam geometry parameters may be predetermined or fixed except for the collimator aperture. Any combination of the beam geometry parameters may be fixed while the remaining beam geometry parameters may be optimised or varied.

For example, the collimator angle may be fixed or predetermined in order to avoid dose bridging. This may be done by choosing a collimator angle so that there is no gap between multiple targets when seen from a beam's eye view and in the direction of movement of the collimator leaves.

The extent to which dose bridging occurs in a particular field depends on the collimator aperture; however, the remaining beam geometry parameters such as couch position or start-stop gantry angle range (or any of the parameters listed above) may be chosen to be optimal so that the collimator aperture may reduce dose bridging more, or most, effectively.

### Bipartite matching

Optionally, the field-target assignments comprise minimum-cost bipartite matching.

In general, minimum-cost bipartite matching divides a set into two subsets, wherein each element (or "vertex") from each subset is connected to every element in the other subset. The "cost" of each connection (or "edge") is determined and then a set of edges that results in the minimum overall cost of the matching is outputted.

Thus:
the bipartite matching uses a complete bipartite graph of vertexes and edges;
a set of vertexes comprises a union of (i) a set of fields and (ii) a set of all non-empty subsets of targets, so that each vertex represents either a field or a targets subset;
each field vertex is connected to all targets subset vertexes, by means of an edge representing one of said assignments;
each edge has an edge cost associated with the corresponding assignment;
   and
the method further comprises finding a set of edges having a minimum sum of edge costs with the constraint that at least one field is assigned to each target.

Bipartite matching is a mathematical algorithm and finding the set of edges having a minimum sum of edge costs, with the constraint that at least one field is assigned to each target, may be solved in a number of ways. Some methods to solve a bipartite matching problem include the Kuhn-Munkres algorithm; the Successive Shortest Path algorithm; the Cost Scaling algorithm; the Primal-Dual algorithm; the Auction algorithm; and the Min-cost Max-flow algorithm.

### Initial collimator leaf aperture

Optionally, said optimising comprises determining an initial collimator leaf aperture for a field by: adjusting a first set of collimator leaves to shape the collimator aperture to cover primary targets to which the field has been assigned; and/or: further determining the initial collimator leaf aperture for the field by: adjusting a second set of collimator leaves disjoint from the first set of collimator leaves, to shape the aperture to cover one or more secondary targets to which the field has not been assigned.

Here, the second set of collimator leaves "disjoint" from the first set of collimator leaves means that none of the leaves of the first set are members of the second set.

Optionally, the method further comprises determining the initial collimator leaf aperture for the field by: adjusting the first set and/or second set of collimator leaves to shape the aperture to cover one or more targets to which the field has not been assigned but which at least partially overlaps the primary targets and/or secondary targets in the beam's eye view of the collimator.

That is, in determining the initial collimator leaf aperture, first the leaf positions are adjusted to fit only the primary targets (that is, the targets to which the field has been assigned). Then, the remaining leaves are adjusted to fit the secondary targets, if possible. By only using the remaining leaves for the secondary targets, no gap is exposed between the primary targets and the secondary targets. In contrast, if the leaves fitted around the primary target were to be moved to accommodate the secondary targets, an undesirable gap may be exposed between the primary target and the secondary target.

After the leaves have been fit around the primary targets and the secondary targets as described above, the positions of the leaves may be further adjusted to include any target tissue that may overlap the primary or secondary target in the beam's eye view of the collimator. That is, the leaf positions are adjusted to include any target or target portion that can be accommodated without incurring dose bridging or irradiation of healthy tissue.

### Treatment plans

Optionally, the plurality of fields corresponds to a plurality of VMAT treatment arcs.

In general, the examples provided in the present disclosure relate to VMAT treatment. However, alternatively, the plurality of fields may correspond to a plurality of "Intensity Modulated Radiation Therapy" (IMRT) fields or VMAT-IMRT hybrid fields. Other radiotherapy treatment types may also be used in accordance with the principles of the present disclosure, wherein the radiotherapy treatment types use a plurality of fields to irradiate a plurality of targets.

Optionally, the method further comprises: dividing one or more of the VMAT treatment arcs into subfields; and assigning each subfield to a different subset, or different proper subset, of targets.

Advantageously, this provides greater flexibility in assigning the fields to the targets, by allowing subfields to be assigned to targets. This in turn allows greater control over the eventual dose distribution in order to avoid dose bridging.

Optionally, the method further comprises generating and optimising a radiotherapy treatment plan based on the plurality of field-target assignments determined for the plan.

For example, after the field-target assignments have been determined, standard dose-based objective function optimisation may be used to generate the final radiotherapy treatment plan. The standard optimisation may be constrained by the field-target assignments determined in accordance with the principles of the present disclosure.

### Other aspects of the invention

The second aspect of the invention comprises a radiation treatment planning computer system comprising a memory and a processor configured to perform the methods as defined above.

The third aspect of the invention comprises a computer program product comprising a non-transitory, computer readable storage medium encoded with instructions operable for execution by a processor to perform the methods as defined above.

According to a fourth aspect of the invention, there is provided means for generating and optimising a radiotherapy treatment plan for treating a plurality of targets using a plurality of fields, each field comprising an optimisable set of control points and each control point comprising a set of beam geometry parameters, comprising:
means for determining a plurality of field-target assignments for the plan by assigning each of the plurality of fields to a respective non-empty subset of the plurality of targets;
wherein at least one field is assigned to each target; and
wherein at least one field is assigned to a non-empty proper subset of the plurality of targets.

The means for generating or optimising a treatment plan and the means for determining the plurality of field-target assignments may comprise the computer system described below.

### BRIEF DESCRIPTION OF THE DRAWINGS

For a better understanding of the present disclosure, some embodiments will now be described by way of example with reference to the accompanying drawings.
Figure 1 shows a block diagram of an example of a computing system upon which some embodiments described herein may be implemented.
Figure 2 is a block diagram showing selected components of a radiation treatment system upon which some embodiments according to the present invention can be implemented.
Figure 3A illustrates elements of an exemplary radiation treatment system upon which radiation treatment plans generated according to some embodiments of the present invention may be implemented.
Figure 3B shows a beam's eye view of a multileaf collimator.
Figure 4 shows a dose distribution achieved in the prior art which involves dose bridging and the irradiation of healthy tissues in between targets.
Figures 5A-5C show three successive (but not consecutive) control points of an exemplary first treatment field or arc, according to the prior art.
Figures 5D-5F show three successive (but not consecutive) control points of an exemplary second treatment field or arc, according to the prior art.
Figure 6 shows a dose distribution that may be achieved under the principles of the present disclosure, which results in reduced dose bridging or reduced irradiation of healthy tissue in between targets.
Figures 7A-7C show three successive (but not consecutive) control points of an exemplary first treatment field or arc, according to principles of the present disclosure.
Figures 7D-7F show three successive (but not consecutive) control points of an exemplary second treatment field or arc, according to principles of the present disclosure.
Figure 8 illustrates the assignment of fields to subsets of a plurality of targets, according to principles of the present disclosure.
Figure 9 is a flowchart for generating a radiotherapy treatment plan using field-target assignments, according to principles of the present disclosure.
Figure 10 illustrates bipartite matching of fields to targets, according to principles of the present disclosure.
Figure 11 is a flowchart for determining a set of field-target assignments associated with a minimal cost.

### DETAILED DESCRIPTION

Reference will now be made in detail to several embodiments. While the subject matter will be described in conjunction with the alternative embodiments, it will be understood that they are not intended to limit the claimed subject matter to these embodiments. On the contrary, the claimed subject matter is intended to cover alternatives, modifications, and equivalents, which may be included within the scope of the claimed subject matter as defined by the appended claims.

Furthermore, in the following detailed description, numerous specific details are set forth in order to provide a thorough understanding of the claimed subject matter. However, it will be recognised by one skilled in the art that embodiments may be practised without these specific details or with equivalents thereof. In other instances, well-known methods, procedures, components, and circuits have not been described in detail as not to unnecessarily obscure aspects and features of the subject matter.

The following description is presented to enable a person skilled in the art to make and use the embodiments of this invention; it is presented in the context of a particular application and its requirements. Various modifications to the disclosed embodiments will be readily apparent to those skilled in the art, and the general principles defined herein may be applied to other embodiments and applications without departing from the scope of the present disclosure. Thus, the present invention is not limited to the embodiments shown, but is to be accorded the widest scope consistent with the principles and features disclosed herein.

### Computer system

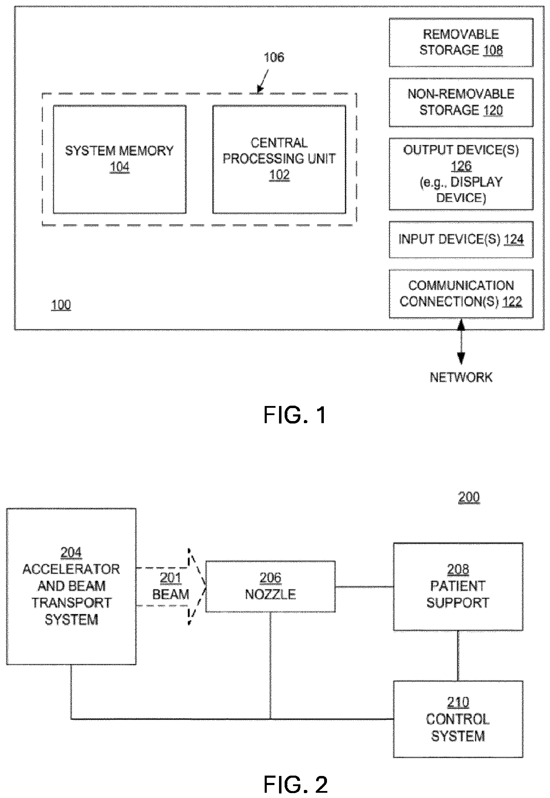

Figure 1 shows a block diagram of an example of a computing system 100 upon which the embodiments described herein may be implemented. In a basic configuration, the system 100 includes at least one processing unit 102 and memory 104. This most basic configuration is illustrated in Figure 1 by dashed line 106. The system 100 may also have additional optional features and/or functionality. For example, the system 100 may also include additional storage (removable and/or non-removable) including, but not limited to, solid state, magnetic or optical disks or tape. Such additional storage is illustrated in Figure 1 by removable storage 108 and non-removable storage 120. The system 100 may also contain communications connection(s) 122 that allow the device to communicate with other devices, e.g., in a networked environment using logical connections to one or more remote computers.

The system 100 also includes input device(s) 124 such as keyboard, mouse, pen, voice input device, touch input device, etc. Output device(s) 126 such as a display device, speakers, printer, etc., are also included.

In the example of Figure 1, the memory 104 includes computer-readable instructions, data structures, program modules, and the like. Depending on how it is to be used, the system 100 - by executing the appropriate instructions or the like - can be used to implement a planning system that generates radiotherapy treatment plans using the methods for field-target assignments disclosed herein.

### Radiation treatment system

Figure 2 is a block diagram showing selected components of a radiation treatment system 200 upon which embodiments according to the present invention can be implemented. In the example of Figure 2, the system 200 includes an accelerator and beam transport system 204 that is operable to generate and/or accelerate a beam 201. The accelerator and beam transport system can generate and deliver beams of various types including, for instance, X-ray (photon) beams. The operations and parameters of the accelerator and beam transport system 204 are controlled so that the intensity, energy, size, and/or shape of the beam are dynamically modulated or controlled during treatment of a patient according to an optimised radiation treatment plan produced by and stored within system 100 as discussed above.

The nozzle 206 is used to aim the beam toward various locations (e.g., of a target) within a patient supported on the patient support device 208 (e.g., a chair, couch, or table) in a treatment room. A target may be an organ, a portion of an organ (e.g., a volume or region within the organ), a tumour, diseased tissue, or a patient outline, for instance.

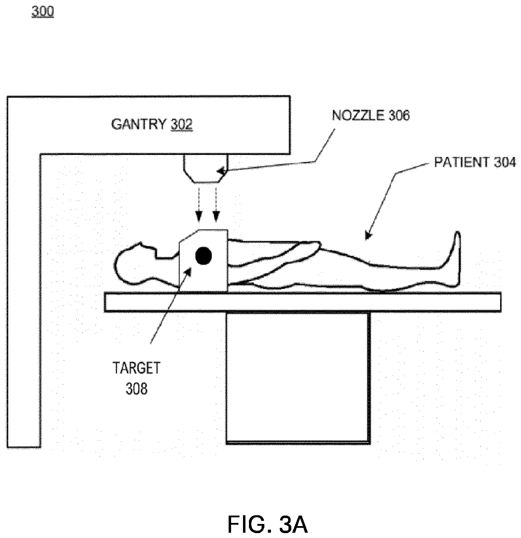

The nozzle 206 may be mounted on or may be a part of a gantry structure (Figure 3A) that can be moved relative to the patient support device 208, which may also be moveable. The accelerator and beam transport system 204 may be mounted on or are a part of the gantry structure; alternatively, the accelerator and beam transport system are separate from (but in communication with) the gantry structure.

The control system 210 of Figure 2 receives and implements a prescribed treatment plan which is generated and/or optimised according to embodiments of the present invention. The control system 210 includes a computing system having a processor, memory, an input device (e.g., a keyboard), and optionally a display; the system 100 of Figure 1 is an example of such a platform for the control system 210. The control system 210 can receive data regarding the operation of the system 200. The control system 210 can control parameters of the accelerator and beam transport system 204, nozzle 206, and patient support device 208, including parameters such as the energy, intensity, size, and/or shape of the beam, direction of the nozzle, and position of the patient support device (and the patient) relative to the nozzle, according to data the control system 210 receives and according to the radiation treatment plan.

Figure 3A illustrates exemplary elements of a radiation treatment system 300 for treating a patient 304. The system 300 is an example of an implementation of the radiation treatment system 200 of Figure 2. The gantry 302 may rotate around an axis, e.g. the gantry may rotate around a patient on a couch. The patient may be moved by moving the couch. While the patient 304 is supine in the example of Figure 3A, the invention is not so limited. For example, the patient 304 can instead be seated in a chair or positioned in any orientation. The gantry 302 can be controlled by a treatment system using an optimised treatment plan generated according to embodiments of the present invention.

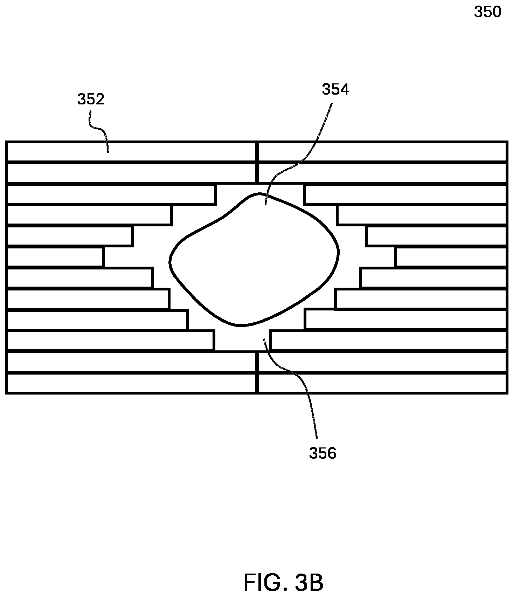

Figure 3B shows a multileaf collimator (MLC) 350 that is part of the radiation treatment system 300. MLCs are used to shape the radiation beam to, for example, conform the beam to the three-dimensional shape of the target or targets to be treated. An MLC comprises a plurality of movable leaves 352 typically made of a high density material such as tungsten and configured to block radiation. The MLC leaves 352 are arranged in two opposing banks, wherein each leaf is independently movable with respect to the other leaves. The two banks of leaves may form an "aperture" 356 through which radiation may pass. By adjusting the position of each leaf, the aperture 356 formed by the leaves may shape the radiation beam to conform to the target's shape 354 or outline in the beam's eye view.

### Dose bridging and collimator apertures

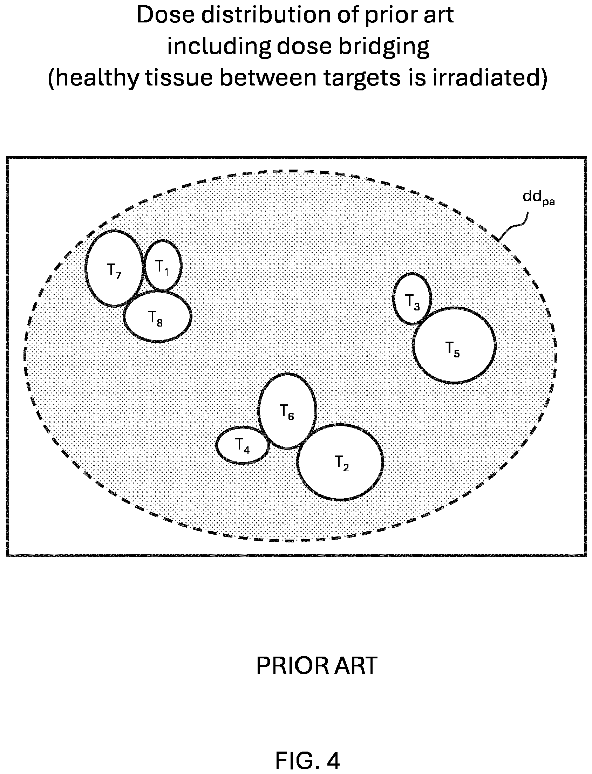

Figure 4 illustrates the dose distribution ddₚₐ achieved by prior art techniques. In this example, there are eight different tumours or targets located in different sites in the patient, that is, targets T₁-T₈. It can be seen that some of the targets are proximate to each other, whilst some of the targets are at a distance from each other. Here, targets T₁, T₇ and T₈ are located proximate to each other in a first group, targets T₃ and T₅ are located proximate to each other in a second group, and targets T₂, T₄ and T₆ are located proximate to each other in a third group. However, the first, second and third groups of targets are located at a distance from each other. A radiation beam that attempts to irradiate all three groups of targets at the same time will generally also irradiate the healthy tissue found between the groups of targets. This is shown by ddₚₐ where in delivering radiation to the targets T₁-T₈ at the same time, the healthy tissue (shown by the shaded region) is also irradiated.

An important part of plan optimisation is that of optimising the positions of the MLC leaves at each control point. In particular, the MLC leaves should form an aperture 356 that conforms to the outline of the target as closely as possible, from a beam's eye view. Iterative optimisation algorithms are typically used to further modulate the leaf aperture within the target outlines to meet the clinical goals as best as possible. This involves determining an initial leaf aperture 356 used at the start of an optimisation for each control point.

A prior art strategy for determining the initial leaf aperture is to set the positions of the leaves so that the radiation beam conformally fits all of the targets (with some margins and/or correction factors). However, attempting to conformally fit the beam to all of the targets by choosing an appropriate aperture might be disadvantageous where there are multiple spatially separate targets, as this might cause the optimiser to provide solutions that involve more dose bridging.

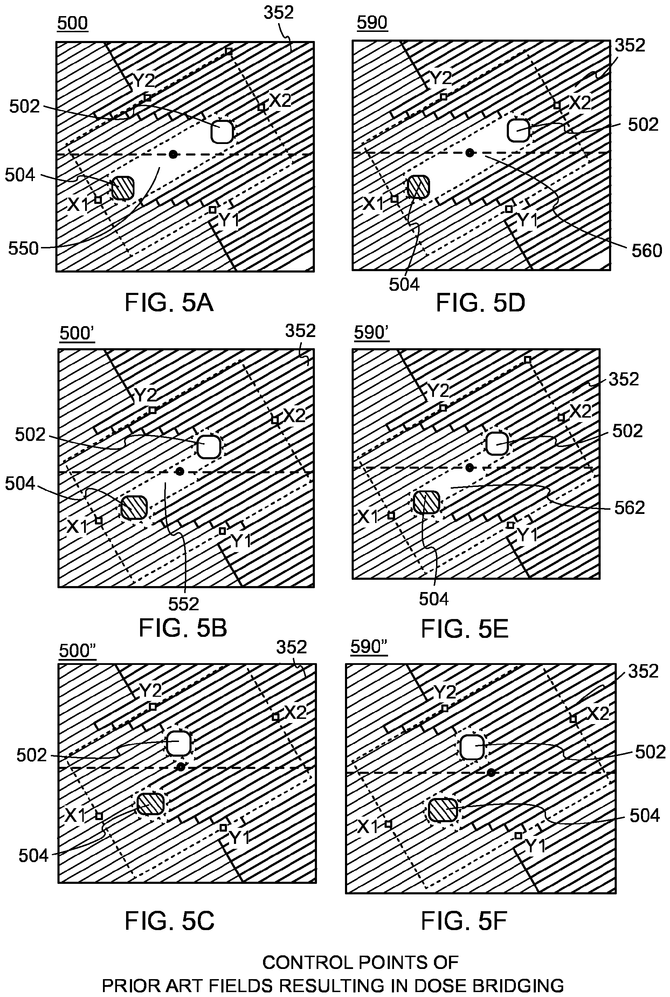

Figures 5A-5F show two targets 502 and 504 from the point of view of a radiation beam. Figures 5A-5C show a first field (or arc), while Figures 5D-5F show a second field (or arc), the first field and the second field relating to the same treatment plan.

Figures 5A-5C show two targets 502 and 504 from the point of view of a radiation beam (i.e., a "beam's eye view") in three successive (but not consecutive) control points of the first field or arc of the treatment plan, in accordance with the prior art. Figures 5A-5C also show the leaves 352 of a multileaf collimator (MLC) that shapes the beam.

Figure 5A shows the initial leaf aperture 550 as might be typical of the prior art. It can be seen that the aperture 550 formed by the MLC leaves 352 will cause the radiation beam to irradiate both targets 502 and 504 in a single (initial) control point 500. However, as the two targets are spatially separate, the radiation beam also irradiates the healthy tissue located between the targets 502 and 504. This happens because the prior art optimiser produces a plan that attempts to treat two spatially separate targets from a single control point.

Figures 5B and 5C show some exemplary successive (but not consecutive) control points 500' and 500" after the initial control point 500, as determined by a plan optimiser of the prior art. It can be seen that substantial dose bridging and irradiation of healthy tissue is taking place in the control point 500', due to the aperture 552 covering a gap between the targets 502 and 504. In the control point 500", it can be seen that dose bridging is not taking place, to illustrate that sometimes the prior art plan optimiser may produce certain control points that do not result in dose bridging (the present disclosure attempts to more consistently produce control points that do not result in dose bridging).

Figures 5D-5F show three successive (but not consecutive) control points of the second field or arc of the same treatment plan, in accordance with the prior art. Figures 5D-5F also show the leaves 352 of an MLC that shapes the beam. Figures 5D-5F are similar to Figures 5A-5C, and it can be seen that the first two illustrated control points 590 and 590' both result in substantial dose bridging and irradiation of healthy tissue due to the apertures 560 and 562. This happens because the plan optimiser is attempting to treat the two spatially separate targets 502 and 504 in the same control point and under the same MLC aperture. As in Figure 5C, the example of Figure 5F shows an MLC aperture that does not produce any dose bridging or irradiation of healthy tissue. This illustrates that some control points in the prior art do achieve reduced dose bridging, but it is the purpose of the present disclosure to reduce dose bridging more consistently and more effectively.

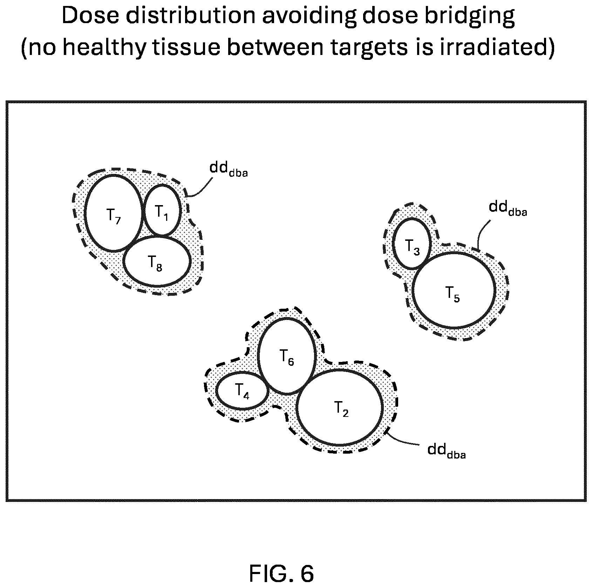

Figure 6 shows an exemplary dose distribution that avoids dose bridging or irradiation of healthy tissue. Figure 6 shows eight exemplary targets T1-T8 that need to be treated by means of a treatment plan. It can be seen that there are three groups of targets comprising a first group of targets T1, T7, T8, a second group of targets T3, T5 and a third group of targets T2, T4, T6. Each group of targets comprise targets that are spatially close to each other, or spatially contiguous. Each group of targets can therefore be treated by means of a single collimator aperture that conforms the radiation beam to the respective group of targets. However, multiple groups of targets, if treated by means of a single collimator aperture, would result in irradiation of healthy tissue in between the multiple groups of targets. This would be the case in the prior art.

By means of the present disclosure, not all fields attempt to treat all targets. This means that some fields or arcs treat only a proper subset of targets. As will be explained in greater detail below, this would result in the reduction of dose bridging, as shown in Figure 6. In the example of Figure 6, there is reduced dose delivered to the healthy tissue in between the first group (T1, T7, T8), the second group (T3, T5) and the third group (T2, T4, T6) of targets. This reduction of dose bridging is one of the aims of the present disclosure.

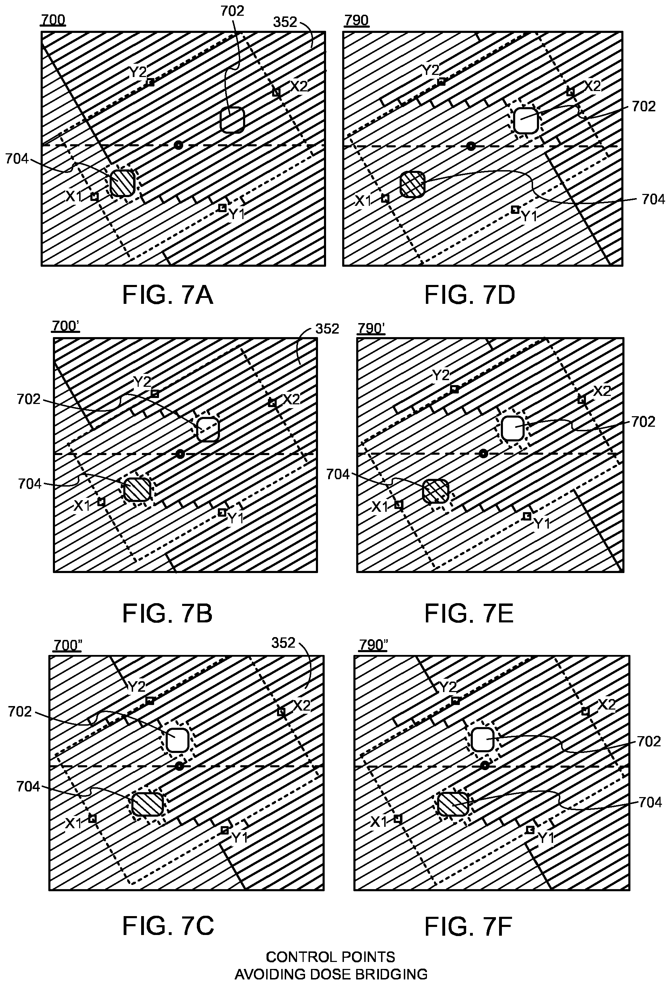

Figures 7A-7F show two targets 702 and 704 from the point of view of a radiation beam. Figures 7A-7C show a first field (or arc), while Figures 7D-7F show a second field (or arc), the first field and the second field relating to the same treatment plan.

Figures 7A-7C show two targets 702 and 704 from the point of view of a radiation beam, i.e., a beam's eye view, similar to Figures 5A-5C. However, while Figures 5A-5C show leaf apertures achieved by the prior art, Figures 7A-7C show exemplary leaf apertures achieved by the present disclosure. Three successive (but not consecutive) control points of a single arc are shown. Figures 7A-7C also show the leaves 352 of the MLC that shapes the beam.

As can be seen, dose bridging is avoided in the example of Figures 7A-7C as there is no healthy tissue that is irradiated in between the targets 702 and 704. This is achieved by assigning each field to a proper subset of targets.

The targets to which a field is assigned are referred to as the "primary targets", and comprise targets that a field is required to irradiate. A field may be assigned to other, "secondary" targets, where secondary targets may, but are not required, to be irradiated by the respective field. In the example of Figures 7A-7C, the field or arc is assigned to a first target 704. Thus, target 704 is a "primary target" for the field or arc of Figures 7A-7C, and target 704 must be treated during the field or arc of Figures 7A-7C.

In this example, target 702 is a "secondary" target, and target 702 may be treated during the field or arc of Figures 7A-7C, but does not have to be treated by the field or arc of Figures 7A-7C. In the exemplary field of Figure 7A-7C, the field is assigned to target 704 as a primary target, but is assigned to target 702 as a secondary target.

At (initial) control point 700, it can be seen that the initial collimator aperture treats only the primary target 704, but does not treat the secondary target 702. That is, the collimator leaves are positioned to allow irradiation of primary target 704, but to block irradiation of secondary target 702. This is because, in accordance with principles of the present disclosure, the optimiser found that it is not possible to irradiate both targets 702 and 704 from control point 700 without also irradiating the healthy tissue between the targets 702 and 704.

At the next exemplary control point 700', the collimator aperture is configured so that the radiation beam treats the primary target 704 and a part of the secondary target 702. Thus, while the aim of the present disclosure is generally to treat only targets to which a field has been assigned, the field may also be assigned to secondary targets which may be irradiated with the primary targets, if the locations of the secondary targets in relation to the primary targets allows this.

This is shown in Figure 7C where both the primary target 704 and the secondary target 702 are irradiated from the same control point. Here, due to the relative position of the primary target 704 and the secondary target 702, both the primary target and the secondary target may be irradiated from the same control point without any dose bridging or irradiation of healthy tissue.

Figures 7D-7F show three successive (but not consecutive) control points of the second field or arc relating to the same treatment plan as the first field or arc shown in Figures 7A-7C. In this example, the field is assigned to target 702 as primary target rather than target 704 as primary target. The field is assigned to target 704 as a secondary target.

At control point 790, only primary target 702 is treated. This is because, in accordance with principles of the present disclosure, the plan optimiser found that it is not possible to treat both primary target 702 and secondary target 704 from the same control point, while avoiding irradiation of healthy tissue located in between primary target 702 and secondary target 704.

At control point 790', the primary target 702 is treated, and a part of the secondary target 704 is also treated. This is because, in accordance with principles of the present disclosure, the plan optimiser found that it is possible to treat primary target 702, and part of secondary target 704, from the same control point without dose bridging.

At control point 790", both the primary target 702 and the secondary target 704 are treated. This is because, in accordance with principles of the present disclosure, the plan optimiser found that it is possible to treat primary target 702 as well as the secondary target 704, from the same control point without dose bridging.

### Field-target assignments

As noted above, in the prior art in general, the plan optimiser attempts to create a radiotherapy treatment plan wherein all the fields aim to treat all the targets, equally. In the prior art, when there are spatially separate targets and yet the plan is developed so that all fields attempt to treat all targets, the resulting plan results in dose bridging. That is, in attempting to irradiate spatially separate targets from the same control point, healthy tissue in between the targets also receives (harmful) radiation.

In contrast, the present disclosure proposes a "multiple fields-multiple targets" technique, that aims to generate better plans for spatially separate targets. That is, each field is assigned to a subset of the targets. At least one of the fields is assigned to a proper subset of the targets.

In an example, a plurality of fields may be assigned to a proper subset of the targets. In another example, many (e.g. >30%) of the fields may be assigned to a proper subset of the targets. In a further example, a majority (e.g. >50%) of the fields may be assigned to a proper subset of the targets. In a still further example, most (e.g. >90%) of the fields may be assigned to a proper subset of the targets.

In an example, the proper subset of the targets may comprise one fewer element than the full set of targets. In another example, the proper subset of the targets may comprise a plurality of fewer elements than the full set of targets. In a further example, the proper subset of targets may comprise many (e.g. >30%) fewer elements than the full set of targets. In a still further example, the proper subset of targets may comprise 50% fewer elements than the full set of targets. In yet another example, the proper subset of targets may comprise 90% fewer elements than the full set of targets.

As noted above, a "field" refers to a set of optimisable control points, each control point relating to a set of beam geometry parameters. By assigning each field to a subset of the targets, each respective field may seek a beam geometry that is most advantageous, in the sense of avoiding dose bridging resulting from treating each respective subset of targets by the respective field. In other words, the field-target assignment of the present disclosure, by allowing each field to treat a different subset of targets, allows the eventual dose distribution provided by all of the fields to conform to the shape of the multiple targets while avoiding healthy tissue in between the multiple targets. This is not possible in scenarios where the optimiser attempts to provide plans wherein all fields treat all of the targets. Thus, this technique is relevant to any plan that has at least two spatially separate targets and has at least two fields or arcs.

For the sake of explanation, consider a treatment that proposes to use n fields or arcs denoted *F = {f_{1,}f₂,...,fₙ},* and m targets *T = {t₁,t₂,...,tₘ}*, with *n*≥2 and *m*≥*2*. According to the present disclosure, the treatment planning optimiser makes an assignment between fields and targets, so that each field only treats the targets to which it is assigned. Targets to which a field has been assigned may be "primary" or "secondary", wherein a "primary" target is a target that the field must irradiate, whereas a secondary target is a target that the field may irradiate if this does not result in, e.g., excessive dose bridging.

According to principles of the present disclosure, the field-target assignment is non-exclusive, meaning that if a field *fᵢ* is assigned to treat a primary target *tⱼ,* the plan optimiser may still generate a plan wherein the field *fᵢ* treats targets other than the primary targets (for example, the plan may irradiate secondary or other targets) if such a plan is superior in achieving the plan objectives.

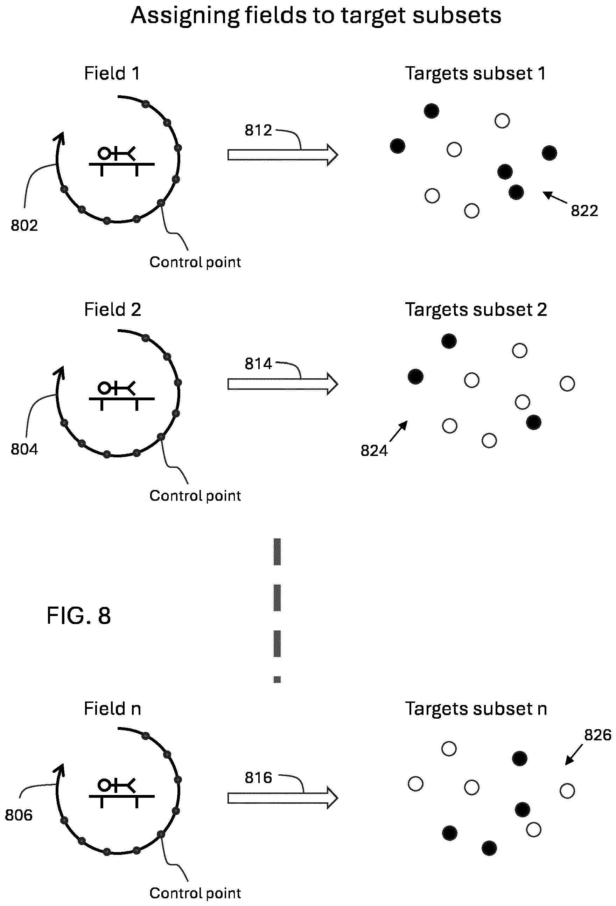

Figure 8 illustrates the field-target assignment of the present disclosure. Figure 8 relates to VMAT therapy and shows, on the left, a plurality of fields, Field 1 - Field n. As explained above, in VMAT, the notion of a "field" refers to a set of optimisable control points, each control point relating to a set of beam geometry parameters such as treatment isocenter, couch position, collimator angle and start-stop gantry angle range. In VMAT, a field comprises a single arc having a set of optimisable control points. Thus, in Figure 8, each partial circle with an arrow at one end represents a field comprising a single VMAT arc. This may be a full arc where the start-stop gantry angle range is 360°, or a partial arc where the start-stop gantry angle range is less than 360°.

Figure 8 shows, on the right, a plurality of target subsets, subset 1 - subset n. Each target is represented by a circle. A target to which a field is assigned is represented by a shaded circle, whereas a target to which the respective field is not assigned is represented by an empty circle. Targets subset 1 has five shaded circles and four empty circles. Field 1 is assigned to Targets subset 1; that is, in this example Field 1 is assigned to a Targets subset comprising five targets. Targets subset 2 has three shaded circles and six empty circles. Field 2 is assigned to Targets subset 2; that is, in this example Field 2 is assigned to a Targets subset comprising three targets. Field n is assigned to Targets subset n; that is, in this example Field n is assigned to a Targets subset comprising four targets.

As shown, each field is assigned to a targets subset. At least one of these subsets is a "proper" subset, in the sense that a proper subset may not include all the targets, but has to be a smaller subset than the full set of targets. By assigning each field to a subset of targets, wherein at least one field is assigned to a proper subset of targets, the resulting dose distribution may be controlled so as to avoid dose bridging.

The method may also use subfields. That is, in some cases of VMAT therapy, an arc field may be divided into subfields prior to the target assignment step. For example, a first half of an arc (first subfield) may be assigned to a first set of targets and a second half of an arc (second subfield) may be assigned to a different set of targets.

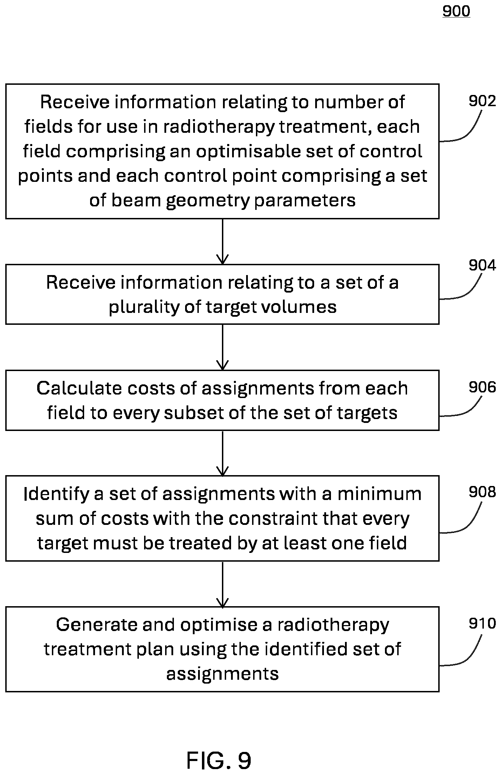

Figure 9 is a flowchart 900 according to an embodiment of the present invention relating to field-target assignment.

In step 902, information is received relating to the number of fields for use in radiotherapy treatment. This number may be predefined, inputted by a human user, or determined by a plan optimiser. In the present method, there are at least two fields in the treatment plan, which in VMAT means there are at least two arcs in the treatment plan.

In step 904, information is received relating to a set of a plurality of target volumes. That is, the present method is applicable in scenarios where there are at least two target volumes. The received information may include the location and number of the target volumes.

In step 906, the costs of hypothetical assignments from each field to every subset of the set of targets is calculated. For example, if there are n fields, there will be n field-target assignments. Further, if there are m targets, then there are 2^{m} possible subsets of targets. A constraint is that every field must be assigned to at least one target, and so that the empty set is excluded from the possible subsets of targets. Thus, when excluding the empty set, there are 2^{m}-1 possible subsets of targets. With n fields and 2^{m}-1 subsets of targets, there are n x (2^{m}-1) possible assignments from the n fields to the 2^{m}-1 subsets of targets, and each possible assignment has associated with it a cost. Methods to calculate the cost of each hypothetical assignment will be described below.

In step 908, a set of field-target assignments having a minimum sum of costs is identified, with the constraint that every target is treated by at least one field.

In step 910, a radiotherapy treatment plan is generated and optimised using the determined set of field-target assignments.

Advantageously, the present method assigns each field or arc to a subset of multiple targets, so that the overall dose distribution obtained by the plurality of fields or arcs avoids dose bridging by locating the overall dose distribution at spatially contiguous targets and not at healthy tissue in between spatially separate targets.

### Calculating the cost of an individual field-target assignment

The cost of assigning a field *fᵢ* to a set of targets *tₐ₁-tₐₖ* may be written mathematically as *C* (*fᵢ*, {*t*_{*a*1}*, t*_{*a*2}, ..., *tₐₖ*}). Here, C may be referred to as a measure of a geometric *suitability cost* for using the field *fᵢ* to treat the *k* targets *t*_{*a*1}*, t*_{*a*2}*,* ..., *tₐₖ.* This cost may be calculated in a number of ways, and some examples include:
i) A standard plan optimiser may be executed using the same objectives as the original plan, but only using the field *fᵢ* and the targets *t*_{*a*1}*, t*_{*a*2}, ... , *tₐₖ.* For example, the standard plan optimiser may use an objective function with conflicting cost terms, and wherein the overall cost of the objective function should be minimised.
   The cost of the optimised plan for only field *fᵢ* and the targets *t*_{*a*1,} *t*_{*a*2}, ... , *tₐₖ,* as calculated using the objective function, may be deemed to be the cost of the field-target assignment C (*fᵢ,* {*t*_{*a*1}*, t*_{*a*2}, ... , *tₐₖ*})*.*
ii) The geometry when treating the targets *t*_{*a*1,} *t*_{*a*2}, ... , *tₐₖ,* using field *fᵢ* may be evaluated by quantifying the overlap between targets and organs at risk from a beam's eye view at each control point of *fᵢ*. These quantities may be combined (e.g. summed) to obtain a cost of the field-target assignment *C* (*fᵢ,* {*t*_{*a*1}*, t*_{*a*2}, ... , *tₐₖ*}).
iii) For each control point in *fᵢ*, a quantity relating to the number of leaves involved in achieving a conformal aperture for the set of targets may be determined. Such quantities across all control points may be combined to obtain a cost of the field-target assignment C (*fᵢ,* {*t*_{*a*1}*, t*_{*a*2}, ... , *tₐₖ*})*.*
iv) At each control point in *fᵢ*, a conformal leaf opening may be generated for the targets *t*_{*a*1,} *t*_{*a*2}*,* ... , *tₐₖ*, and a measure of the amount of healthy tissue that gets exposed in the opening and that does not overlap with any target may be quantified. Such a measure across all the control points may be combined (e.g. summed) and the combined measure may be regarded as a cost of the field-target assignment C (*fᵢ,* {*t*_{*a*1}*, t*_{*a*2}, ... , *tₐₖ*}).
v) At each control point in *fᵢ*, a distance from the targets to the source of radiation may be determined. These distances may be combined (e.g. summed) across control points to determine the cost of the field-target assignment *C* (*fᵢ,* {*t*_{*a*1}*, t*_{*a*2}, ... , *tₐₖ*}).
vi) At each control point in *fᵢ*, a conformal leaf opening may be generated for the targets *t*_{*a*1,} *t*_{*a*2}, ... , *tₐₖ,* and a measure of the distance that the leaves need to travel to achieve such an opening may be quantified. Such a measure across all the control points may be combined (e.g. summed) and the combined measure may be regarded as a cost of the field-target assignment *C* (*fᵢ,* {*t*_{*a*1}*, t*_{*a*2}, ... , *tₐₖ*}).

The cost or suitability of assigning any particular field to any particular subset of targets may be determined using any of the above methods, and this may be used to determine the optimal set of field-target assignments as discussed below.

### Describing assignments using set theory

The cost associated with each possible, hypothetical assignment of every field to every possible subset of targets may be denoted by: $C \left(F\times {P}^{+}\left(T\right)\right) \to ℝ$ ${P}^{+} \left(T\right) = P \left(T\right) \ \left\{∅\right\}$ here:
C = cost
F = Set of fields
*x* = cross product
T = Set of targets
P(T) = power set of T, i.e., set of all subsets of targets
+ = non-empty set
\0 = exclude empty sets
P⁺(T) = power set comprising all non-empty subsets of targets
$ℝ = set of real numbers$

The notion of a set of all subsets is referred to as a power set and is denoted P(T), in accordance with the symbol used in conventional set theory. A set of all subsets includes every possible combination of subsets, including the empty set. For example, a set of 3 targets E1, E2 and E3 will have a power set comprising the following set of targets subsets (without listing the empty subset): {E1}; {E2}; {E3}; {E1,E2}; {E1, E3}; {E2, E3}; {E1, E2, E3}.

A non-empty set is denoted by the symbol +, in accordance with conventional set theory. Thus, the equation 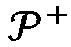 (*T*) *=* 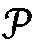 (*T*) \{Ø} mathematically describes the power set of the set of targets T without the empty subset (see Equation 2).

Every assignment of every field to every element in the powerset P⁺(T) may be mathematically written as the cross product of the two sets F and P⁺(T), i.e., F x P⁺(T). Each assignment of every field to every element in the powerset P⁺(T) has associated with it a cost, denoted by C in Equation 1.

### Bipartite graph

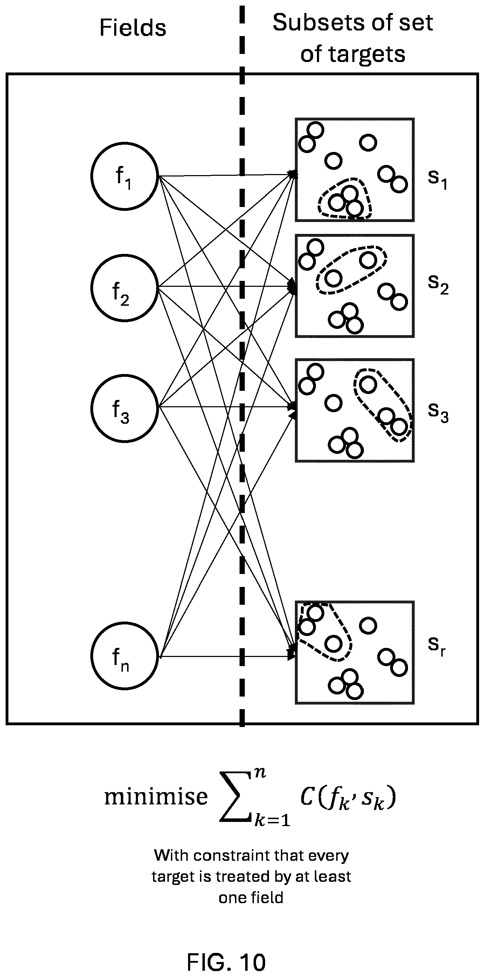

The problem of finding the optimal set of assignments from each field to a respective subset of targets can be formulated as a combinatorial problem in a bipartite graph. Figure 10 illustrates a bipartite graph between a set of fields f₁-fₙ and a set of non-empty subsets of targets s₁-sᵣ. As can be seen, in the bipartite graph of Figure 10 every field fᵢ is connected to every possible set of subsets sᵣ of targets. Every such connection has associated with it a cost - for example, a connection resulting in excessive dose bridging may have a higher cost. By taking into account the cost of every possible assignment, a final set of assignments is determined so that the overall cost is minimised.

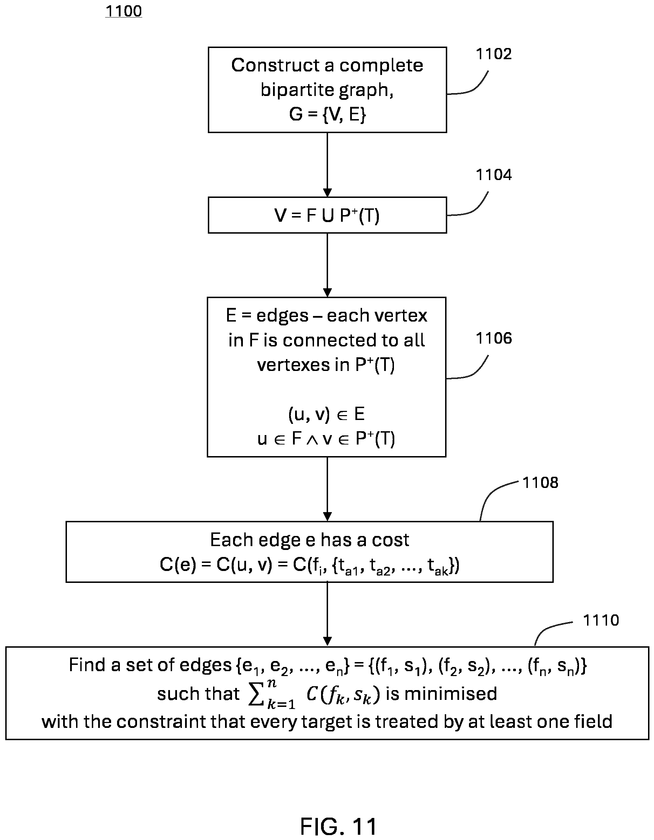

The above steps may be described using minimum-cost bipartite matching, as illustrated in Figure 11 in conjunction with Figure 10. First, a complete bipartite graph is constructed 1102. A complete bipartite graph may be written as: $G = \left\{V, E\right\}$

That is, a bipartite graph G comprises vertexes V and edges E, each edge connecting two vertexes.

Then: $V = F U {P}^{+} \left(T\right)$

That is, the set of all vertexes V comprises the union of (i) the set of all fields F (i.e., f₁-fₙ) and (ii) the set of all non-empty subsets P⁺ of the set of targets T (i.e., s₁-sᵣ), see step 1104 of Figure 11.

Then: $\left(u, v\right) ∈ E ⇔ u ∈ F ∧ v ∈ {P}^{+} \left(T\right)$ here:
u is a field and is an element of the set of fields F
v is a subset of the set of targets and is an element of the set of all non-empty subsets of the set of targets T
(u, v) is an edge between field u and target subset v, and is an element of the set of edges
⇔ means that node u,v are connected by an edge if and only if u represents a field, and v represents a subset of the targets.

That is, field u assigned to a subset v of targets is an element of the set of edges E. This is shown in step 1106 of Figure 11.

Then: $C \left(e\right) = C \left(u, v\right) = C \left({f}_{i}, \left\{{t}_{a 1},{t}_{a 2},…,{t}_{ak}\right\}\right)$

Thus, every edge e (which represents an assignment of a field u to a subset of targets v) has a cost associated with it. This is shown in step 1108 of Figure 11.

Then the optimisation process attempts to find a set of edges: $\left\{{e}_{1},{e}_{2},…,{e}_{n}\right\} = \left\{\left({f}_{1}, {s}_{1}\right),\left({f}_{2}, {s}_{2}\right),…,\left({f}_{n}, {s}_{n}\right)\right\}$ such that: ${∪}_{k = 1}^{n} {s}_{k} = T$ such that: ${\sum }_{k = 1}^{n} C \left({f}_{k}, {s}_{k}\right)$ is minimised.

In the above equations:
e = edge
n = number of edges / number of fields
s = a subset of the set of all targets
Uⁿₖ₌₁ sₖ = union of all subsets used in the mapping
T = set of all targets
k = mapping index
C(fₖ, sₖ) = cost of field k assigned to subset k of targets

That is, when field k is assigned to targets subset k, this is denoted as an edge k, and would have an associated cost C(fₖ, sₖ). The sum of the costs of all such assignments needs then to be minimised. This is shown in step 1110 of Figure 11. In other words, the problem would be reduced to finding an assignment from each of the fields into a respective subset of the targets, such that all the targets are treated by at least by one field, and such that the total cost is minimised. This can be seen as a variation of the concept of "minimum-cost bipartite matching" which may be solved, for example, in polynomial time using algorithms such as the Kuhn-Munkres algorithm; the Successive Shortest Path algorithm; the Cost Scaling algorithm; the Primal-Dual algorithm; the Auction algorithm; and the Min-cost Max-flow algorithm.

### Non-exclusivity

The above-described method may be used to assign each field to a subset of targets, wherein the subset of targets to which a field is assigned may be referred to as "primary" targets. However, during optimisation the fields are not necessarily restricted to irradiating the primary targets, but may also irradiate other targets referred to as "secondary" targets. This may happen when it is possible to irradiate the primary targets as well as one or more secondary targets whilst still avoiding or reducing dose bridging between the primary targets and the secondary targets.

Similarly, the plan may be optimised so that a field may irradiate a target that is neither a primary nor a secondary target, but which can still be irradiated without causing dose bridging.

### Beam geometry for each field-target assignment

Prior to calculating the cost of a field-target assignment, the beam geometries of the respective field are optimised as if the field is treating only its primary targets. For example, the isocenter, couch angle, start-stop gantry range or collimator angle of a field may be optimised for the primary targets to which the field has been assigned. The cost of a field-target assignment is then calculated based on the optimal beam geometry for the particular field and the subset of targets. In the bipartite matching described above, the cost of each field-target assignment, as calculated under the optimal beam geometry, may be associated with each edge between each field and the subset of targets that the field has been assigned to.

When determining the optimal beam geometry for each field-target assignment, beam geometry parameters comprising the collimator angle, the couch angle, and isocenter can be optimised so that they have the same value for a particular arc, or alternatively, so that their value changes over the course of the arc.

Once the problem of matching each field with a subset of targets is solved, the optimal beam geometries used to calculate the cost of each field-target assignment may be used to treat the patient. Optionally, after the optimal (lowest cost) field-target assignment has been determined, conventional dose-based optimisation may be performed in order to generate the final radiotherapy treatment plan. In this case, the dose-based optimisation would be constrained by the field-target assignment generated in accordance with principles of the present disclosure.

### Determining initial collimator apertures

The collimator apertures of a particular field or arc may be initialised with the following steps. The positions of a first set of collimator leaves are adjusted to shape the collimator aperture to cover only the primary targets to which the field has been assigned. Then, the positions of a second set of collimator leaves, disjoint from the first set of collimator leaves, are adjusted to shape the aperture to cover secondary targets to which the field has not been assigned. Then, the positions of the first set and/or the second set of collimator leaves may be further adjusted to cover one or more targets to which the field has not been assigned, but which at least partially overlaps the primary targets and/or the secondary targets. This method allows the leaf positions to expand to cover as many of the primary, secondary and other targets, but to reduce the exposed area between the targets.

Embodiments of the present invention are thus described. While the present invention has been described in particular embodiments, it should be appreciated that the present invention should not be construed as limited by such embodiments, but rather construed according to the following claims.

## Claims

1. A method for generating and optimising a radiotherapy treatment plan for treating a plurality of targets using a plurality of fields, each field comprising an optimisable set of control points and each control point comprising a set of beam geometry parameters;
wherein:
the method comprises determining a plurality of field-target assignments for the plan by assigning each of the plurality of fields to a respective non-empty subset of the plurality of targets;
at least one field is assigned to each target; and
at least one field is assigned to a non-empty proper subset of the plurality of targets.

2. The method of claim 1, further comprising calculating a cost associated with each possible assignment of each of the plurality of fields to a plurality of non-empty subsets of the plurality of targets, and wherein the plurality of field-target assignments for the plan comprises a set of assignments having a minimum sum of associated costs.

3. The method of claim 1 or claim 2, wherein each of said plurality of field-target assignments for the plan is non-exclusive so that during optimisation of the plan, targets to which a field was not assigned may be planned to be treated by the field.

4. The method of claim 2, wherein the cost associated with an assignment of a field to a non-empty subset of targets can be computed by one or more of:
(i) a cost of a plan generated by executing a plan optimiser using plan objectives while restricting the plan to said field and said non-empty subset of targets;
(ii) a quantification of an overlap between targets and organs at risk under the assignment;
(iii) a number of collimator leaves involved in the assignment;
(iv) an amount of healthy tissue exposed in a collimator aperture covering all targets in the non-empty subset of targets;
(v) distance from a source of radiation to the non-empty subset of targets; and
(vi) distance from the non-empty subset of targets to the center of an isocenter plane, on an axis parallel to leaf direction.

5. The method of any preceding claim, wherein the beam geometry parameters comprise one or more of:
multileaf collimator leaf positions;
collimator angle;
treatment isocenter;
couch position; and
start-stop gantry angle range.

6. The method of any preceding claim, wherein the beam geometry parameters are optimised to be static during a gantry rotation;
or:
wherein the beam geometry parameters are optimised to be dynamic during a gantry rotation.

7. The method of any preceding claim wherein the assignments comprise minimum-cost bipartite matching.

8. The method of claim 7, wherein:
the bipartite matching uses a complete bipartite graph of vertexes and edges;
a set of vertexes comprises a union of (i) a set of fields and (ii) a set of all non-empty subsets of targets, so that each vertex represents either a field or a targets subset;
each field vertex is connected to all targets subset vertexes, by means of an edge representing one of said assignments;
each edge has an edge cost associated with the corresponding assignment;
and
the method further comprises finding a set of edges having a minimum sum of edge costs with the constraint that at least one field is assigned to each target.

9. The method of any preceding claim, wherein said optimising comprises determining an initial collimator leaf aperture for a field by:
adjusting a first set of collimator leaves to shape the collimator aperture to cover primary targets to which the field has been assigned;
and/or:
further determining the initial collimator leaf aperture for the field by:
adjusting a second set of collimator leaves disjoint from the first set of collimator leaves, to shape the aperture to cover one or more secondary targets to which the field has not been assigned.

10. The method of claim 9, further comprising determining the initial collimator leaf aperture for the field by:
adjusting the first set and/or second set of collimator leaves to shape the aperture to cover one or more targets to which the field has not been assigned but which at least partially overlaps the primary targets and/or secondary targets.

11. The method of any preceding claim, wherein the plurality of fields corresponds to a plurality of VMAT treatment arcs.

12. The method of claim 11 further comprising:
dividing one or more of the VMAT treatment arcs into subfields; and
assigning each subfield to a different subset of targets.

13. The method of any preceding claim further comprising generating and optimising a radiotherapy treatment plan based on the plurality of field-target assignments determined for the plan.

14. A radiation treatment planning computer system comprising a memory and a processor configured to perform the method as defined in any one of claims 1-13.

15. A computer program product comprising a non-transitory, computer readable storage medium encoded with instructions operable for execution by a processor to perform the method as defined in any one of claims 1-13.
